# EUROPÄISCHE PATENTSCHRIFT

(11) **EP 1 254 632 B1**
(45) Veröffentlichungstag und Bekanntmachung des Hinweises auf die Patenterteilung: **26.07.2006**
(21) Anmeldenummer: 02008685.6
(22) Anmeldetag: 18.04.2002
(51) Int. Cl.: A61B 5/15

(54) **Blutentnahmesystem mit Schwenkgelenk**
Blood lancet with pivot mechanism
Lancette de prélèvement sanguin avec un mécanisme de pivot

(30) Priorität: 05.05.2001 DE 10121883
(43) Veröffentlichungstag der Anmeldung: 06.11.2002
(73) Patentinhaber: Roche Diagnostics GmbH, 68298 Mannheim (DE); F. HOFFMANN-LA ROCHE AG, 4070 Basel (CH)
(72) Erfinder: List, Hans, 64754 Hesseneck-Kailbach (DE)
(74) Vertreter: Pfeifer, Hans-Peter

(56) Entgegenhaltungen:
- US-A- 5 997 561
- US-A- 6 071 249

## Beschreibung

Um für analytisch-diagnostische Zwecke eine geringe Menge Blut aus einem Körperteil (üblicherweise dem Finger oder dem Ohrläppchen) zu entnehmen, werden Lanzetten verwendet, die in das entsprechende Körperteil gestochen werden. Soweit die Lanzetten zur Erzeugung einer Wunde manuell in die Haut gestochen werden, ist speziell trainiertes Personal erforderlich. Dennoch ist der Einstich mit einem erheblichen Schmerz verbunden.

Bereits seit langem werden Blutentnahmesysteme verwendet, die aus einem Stechgerät und zugehörigen, für das jeweilige Gerät speziell angepaßten Lanzetten bestehen. In einem Gehäuse des Stechgerätes ist ein Lanzettenantrieb enthalten, durch den eine Lanzette mechanisch in die Haut eingestochen wird. Als Antriebselement für die Einstichbewegung dient in der Regel eine Feder. In der Anfangsphase der Entwicklung waren sehr einfache Konstruktionen gebräuchlich, bei denen die Lanzette unmittelbar an einem Ende einer in einem länglichen Gehäuse angeordneten Druckfeder befestigt war (z.B. US-Patent 4,469,110).

Derartige Blutentnahmesysteme wurden jedoch den hohen Anforderungen nicht gerecht, die zu erfüllen sind, wenn eine regelmäßige Überwachung bestimmter analytischer Werte des Blutes erforderlich ist. Dies gilt insbesondere für Diabetiker, die ihren Blutzuckerspiegel häufig kontrollieren sollten, um durch Anpassung von Insulininjektionen an den Bedarf (der abhängig von der Nahrungsaufnahme, der körperlichen Aktivität etc. stark schwankt) ihren Blutzuckerspiegel möglichst ständig innerhalb bestimmter Sollgrenzen zu halten. Durch umfangreiche wissenschaftliche Untersuchungen wurde bewiesen, daß mittels einer Intensivtherapie mit mindestens vier Blutanalysen pro Tag ein dramatischer Rückgang schwerster Spätschäden des Diabetes Mellitus (beispielsweise einer Retinopathie mit resultierender Erblindung des Patienten) erreicht werden kann.

Eine solche Intensivtherapie setzt jedoch voraus, daß die Blutentnahme mit einem möglichst geringen Schmerz verbunden ist. Es wurden deshalb zahlreiche Blutentnahmesysteme entwickelt, die diesbezüglich eine Verbesserung bewirken sollten. Beispiele dieser Entwicklung sind in den nachfolgend diskutierten Druckschriften beschrieben.

Bei der in dem US-Patent 4,924,879 beschriebenen Konstruktion wirkt eine Spiral-Antriebsfeder auf ein Rad, dessen Drehung mittels einer Pleuelstange in eine Einstich- und Rückführbewegung der Lanzette umgesetzt wird. Der Schmerz soll dadurch vermindert werden, daß diese Bewegung sehr schnell abläuft. Die Konstruktion unter Verwendung präzise gefertigter Metallteile ist jedoch aufwendig und relativ voluminös. Außerdem ist nachteilig, daß die Lanzette beim Spannen des Lanzettenantriebs aus der Austrittsöffnung austritt und daraus eine Verletzungsgefahr resultiert.

In dem US-Patent 4,924,879 ist eine Konstruktion beschrieben, bei der die erforderliche Verbindung zwischen einem (als Blattfeder oder Spiralfeder ausgebildeten) Antriebselement und der Lanzette von zwei über ein Schwenkgelenk miteinander verbundenen Hebeln gebildet wird. Einer der Hebel ist an seinem der Lanzette zugewandten Ende über ein zweites Schwenkgelenk mit der Lanzette verbunden, während der andere Hebel über ein drittes Schwenkgelenk mit dem Gehäuse verbunden ist. Die Schwenkachsen aller drei Schwenkgelenke laufen parallel. Im gespannten Zustand befindet sich der von den beiden Hebeln gebildete Kniehebel in einer ersten geknickten Position. Nach der Betätigung eines Auslösers wird er unter der Wirkung der Antriebsfeder über eine gestreckte Position in eine zweite geknickte Position bewegt, in der sich das erste Schwenkgelenk, bezogen auf die Ausgangsposition, auf der anderen Seite einer durch den Einstichweg der Lanzette und die Achse des zweiten Schwenkgelenkes definierten Ebene befindet. Auch bei dieser Konstruktion tritt die Lanzette beim Spannen aus der Austrittsöffnung aus.

Blutentnahmesysteme mit dem in dem US-Patent 5,318,584 beschriebenen Lanzettenantrieb werden in großem Umfang verwendet und von den Benutzern wegen ihrer unübertroffenen Schmerzarmut sehr geschätzt. Kernelement dieses Antriebs ist ein Rotorteil, dessen Rotorachse mit der Längsachse des langgestreckten ("Pencil-förmigen")-Gerätes übereinstimmt. Dieses Rotorteil wird über eine koaxiale Spiralfeder angetrieben. Seine Rotationsbewegung wird über eine Kurvensteuerung in die erforderliche Translationsbewegung der Lanzette übertragen. Die Gestaltung der Steuerkurve ermöglicht es, das Gerät zu spannen, ohne daß die Lanzettenspitze aus dem Gehäuse austritt. Die Rotation des Rotorteils um die Längsachse des Gerätes führt zu einer hohen Vibrationsarmut und stabilisiert den Einstichvorgang. Die Konstruktion besteht jedoch aus relativ vielen kompliziert geformten Teilen und ist deswegen aufwendig. Eine neuere Version einer Blutentnahmesystem mit einem um die Gerätelängsachse rotierenden Rotorteil ist in der EP 1034740 A1 beschrieben.

In der EP 1090584 A2 ist eine weitere Ausgestaltung des Rotorprinzips beschrieben, bei der ein verminderter konstruktiver Aufwand durch Verwendung eines speziell geformten Antriebsrotors erreicht wird, wobei die Drehung des Rotors dadurch bewirkt wird, daß die Kraft der Antriebsfeder gegen eine entsprechend geformte Druckfläche des Antriebsrotors drückt. Auch in diesem Fall wird die Bewegung des Antriebsrotors mittels einer Kurvensteuerung in eine entsprechende Bewegung der Lanzette umgewandelt. Die Konstruktion bedingt eine relativ breite Gehäuseform, die von vielen Benutzern als ungünstig angesehen wird.

Trotz der umfangreichen Entwicklungsarbeiten, die zu den vorstehend erörterten und zahlreichen weiteren Konstruktionen geführt haben, besteht ein großes Interesse an einem Blutentnahmesystem, das die teilweise gegenläufigen Anforderungen
- minimales Schmerzempfinden,
- möglichst einfache Bedienung,
- kompakte schlanke Bauweise und
- einfache, kostengünstige Konstruktion
gleichzeitig möglichst weitgehend erfüllt.

Ein wesentlicher Fortschritt in dieser Hinsicht wird mit einem erfindungsgemäßen Blutentnahmesystem zur Entnahme von Blut für Diagnosezwecke erreicht, das ein Gehäuse mit einer Austrittsöffnung für die Spitze einer Lanzette, die in dem Gehäuse entlang einem vorbestimmten Einstichweg beweglich ist, eine Lanzettenführung, von der die Lanzette auf dem vorbestimmten Einstichweg geführt wird und einen Lanzettenantrieb, durch den nach Betätigung eines Auslösers eine Bewegung eines Antriebselementes in eine Einstichbewegung umgesetzt wird, bei der die Lanzette mit hoher Geschwindigkeit entlang dem vorbestimmten Einstichweg in Einstichrichtung bewegt wird, bis ihre Spitze aus der Austrittsöffnung austritt, umfaßt. Der Lanzettenantrieb weist einen Mehrhebel-Kopplungsmechanismus auf, der während der Einstichbewegung eine Verbindung zwischen dem Antriebselement und der Lanzette bildet und zwei Hebel einschließt, die über ein erstes Schwenkgelenk miteinander verbunden sind. Einer der Hebel ist an seinem der Lanzette zugewandten Ende über ein zweites Schwenkgelenk mit der Lanzette gekoppelt. Der zweite Hebel weist an seinem von der Lanzette abgewandten Ende ein drittes Schwenkgelenk auf. Der Mehrhebel-Kopplungsmechanismus wird während der Einstichbewegung aus einer dem gespannten Zustand des Lanzettenantriebs entsprechenden ersten geknickten Position über eine der maximalen Einstichtiefe entsprechende gestreckte Position in eine dem entspannten Zustand des Lanzettenantriebs entsprechende zweite geknickte Position bewegt. Während einer Spannbewegung wird er von der zweiten geknickten Position über die gestreckte Position in die erste geknickte Position bewegt. Die Bewegungsfreiheit der Lanzette ist während der Spannbewegung derartig limitiert, daß die Lanzettenspitze nicht aus der Austrittsöffnung austritt. Das dritte Schwenkgelenk ist an einem beweglichen Lagerteil befestigt, das in dem Gehäuse während des Spannvorgangs eine Bewegung entgegen der Einstichrichtung ausführt und dessen Bewegungsfreiheit während der Einstichbewegung entgegen der Einstichrichtung limitiert ist.

Entgegen der beschriebenen jüngeren Entwicklung greift die Erfindung auf die in der EP 0458451 A1 beschriebene Konstruktion zurück, bei der die Verbindung zwischen der Antriebsfeder (oder einem anderen geeigneten Antriebselement) und der Lanzette durch zwei Hebel gebildet wird, die (nach Art eines Kniehebels) über Schwenkachsen miteinander und mit der Lanzette verbunden sind.

Der Begriff "Hebel" bezeichnet im Rahmen der vorliegenden Erfindung ein Bauelement, das starr ist und eine Verbindung zwischen zwei jeweils als Schwenklager ausgebildeten Kraft-Angriffspunkten bildet. Die in der EP 0458451 A1 dargestellte, zwischen den Schwenkachsen stabförmige Gestaltung der Hebel ist nur eine von mehreren Möglichkeiten. Für die Realisierung der Hebel-Bauteile kommen auch andere Formen in Betracht, wie noch näher erläutert wird.

Offenbarungen US-A-5 997 561 und US-A-6 071 249 beschreiben Limitierungen der Einstickbewegung. Dadurch tritt die Lanzettenspitze aus der Austrittsöffnung nicht aus, aber diese Limitierungen sind ohne mehrhebelkopplungsmechanismus.

Im Rahmen der Erfindung unterliegen sowohl die Lanzette als auch das Lagerteil, an dem das hintere Ende des Kopplungsmechanismus angelenkt ist, während der unterschiedlichen Bewegungsphasen des Lanzettenantriebs unterschiedlichen Bewegungsrestriktionen.
- Während der Spannbewegungsphase ist die Lanzette, vorzugsweise mittels des ohnehin vorhandenen Auslösers, in Einstichrichtung so limitiert, daß ihre Spitze nicht aus der Austrittsöffnung des Gehäuses austreten kann. Dadurch wird die Verletzungsgefahr vermieden. Andererseits ist das Lagerteil, an dem das dritte Schwenkgelenk befestigt ist, nach hinten (also entgegen der Einstichrichtung) beweglich und ermöglicht dadurch die beim Spannen erforderliche Streckung des Kopplungsmechanismus.
- Während der Einstichbewegungsphase ist die Lanzette frei, um die Einstichbewegung durchführen zu können. Die Bewegungsfreiheit des Lagerteils nach hinten hingegen ist in dieser Phase zumindest insoweit limitiert, wie dies erforderlich ist, um das bei der Einstichbewegung erforderliche Austreten der Lanzettenspitze aus der Austrittsöffnung sicherzustellen.

Die praktische Erprobung der Erfindung hat ergeben, daß durch diese Kombination von Maßnahmen eine Mehrhebel-Kopplungsmechanik, die bisher nur in anspruchslosen zur einmaligen Verwendung vorgesehenen "Einwegsystemen" praktisch verwendet wurde, in einem hochwertigen Produkt mit sehr positiven Eigenschaften hinsichtlich der vorstehend erläuterten Anforderungen eingesetzt werden kann.

Die Erfindung wird nachfolgend anhand von in den Figuren dargestellten Ausführungsbeispielen näher erläutert. Die darin beschriebenen Merkmale können einzeln oder in Kombination verwendet werden, um bevorzugte Ausgestaltungen der Erfindung zu schaffen. Es zeigen:
- Fig. 1: Eine Längsschnitt-Prinzipdarstellung eines erfindungsgemäßen Blutentnahmesystems in vier unterschiedlichen Bewegungspositionen (a) bis (d) des Lanzettenantriebs,
- Fig. 2: eine Aufsicht auf das Stechgerät eines zur praktischen Erprobung der Erfindung verwendeten Blutentnahmesystems,
- Fig. 3: einen Längsschnitt durch das in Fig. 2 dargestellte Gerät entlang der Linie A-A,
- Fig. 4: einen Teil-Längsschnitt durch das in Fig. 2 dargestellte Gerät entlang der Linie B-B,
- Fig. 5: eine Längsschnitt-Prinzipdarstellung von einer alternativen Ausführungsform eines erfindungsgemäßen Blutentnahmesystems in drei Bewegungspositionen (a) bis (c),
- Fig. 6: eine Darstellung analog Figur 1 mit einer alternativen Ausführungsform des Lanzettenantriebs.

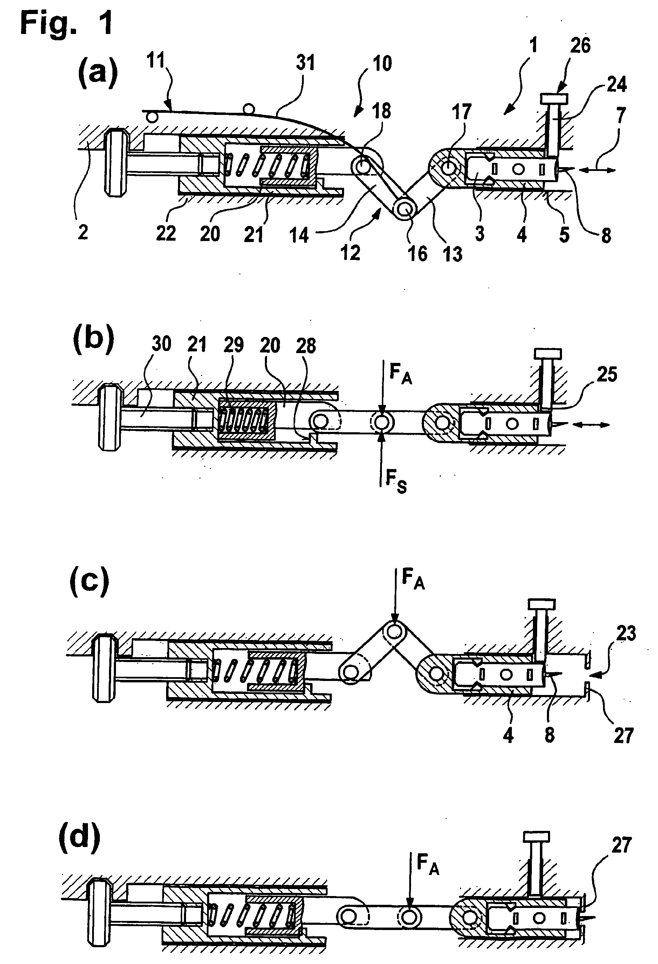

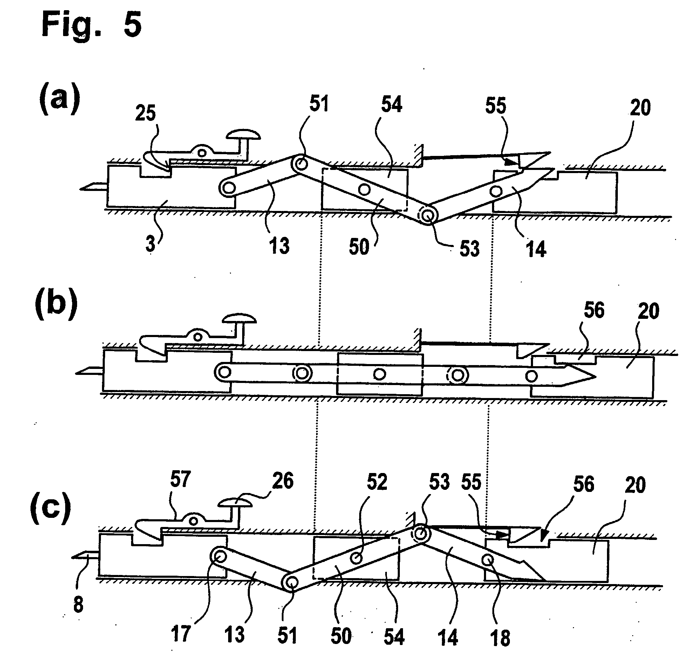

Das Blutentnahmesystem 1 besteht aus einem (in den Figuren 1 und 5 nur partiell dargestellten) Stechgerät 2 und Lanzetten 3. Bei den dargestellten Ausführungsformen ist die Lanzette 3 jeweils auswechselbar in einem Lanzettenhalter 4 fixiert, der seinerseits von einer Lanzettenführung 5 bei der Einstichbewegung der Lanzette auf einem vorbestimmten Einstichweg 7 geführt ist. Die Führung der Lanzette 3 auf dem Einstichweg 7 erfolgt hier also indirekt über den Lanzettenhalter 4. Die Erfindung ist jedoch auch in Verbindung mit "direktgeführten" Lanzetten verwendbar, die während der Einstichbewegung lediglich an ihrem von der Lanzettenspitze 8 abgewandten Ende mit dem Lanzettenantrieb des Stechgerätes gekoppelt sind und unmittelbar (insbesondere durch die sie umgebende Gehäusewandung) geführt werden.

Die Lanzette 3 ist (bei der dargestellten Ausführungsform indirekt über den Lanzettenhalter 4) während der Einstich- und Rückführbewegung mit einem Lanzettenantrieb 10 gekoppelt, der im wesentlichen aus einem Antriebselement 11 und einem Kraftübertragungs-Kopplungsmechanismus besteht, der bei der Erfindung ein Mehrhebel-Kopplungsmechanismus 12 ist. Der Mehrhebel-Kopplungsmechanismus 12 besteht bei der in den Figuren 1 bis 4 dargestellten Ausführungsform aus zwei Hebeln, nämlich einem lanzettenseitigen Hebel 13 und einem antriebsseitigen Hebel 14, die nach Art eines Kniehebels über ein erstes Schwenkgelenk 16 schwenkbar miteinander verbunden sind. Der lanzettenseitige Hebel 13 ist an seinem der Lanzette 3 zugewandten Ende über ein zweites Schwenkgelenk 17 und über den Lanzettenhalter 4 mit der Lanzette 3 gekoppelt. Der antriebsseitige Hebel 14 ist an seinem von der Lanzette 3 abgewandten Ende über ein drittes Schwenkgelenk 18 mit einem Lagerteil 20 verbunden, das mittels einer Lagerteilführung 21 in dem Gehäuse 22 des Stechgerätes 2 derartig beweglich gelagert ist, daß es eine Bewegung entgegen der Einstichrichtung (d.h. eine Bewegung, die mindestens eine Komponente entgegen der Einstichrichtung hat) ausführen kann. Die Schwenkachsen der Schwenkgelenke 16 bis 18 verlaufen parallel zueinander senkrecht zu der Zeichenebene der Figuren.

Bei der in Figur 1 dargestellten Ausführungsform wird die erforderliche Limitierung der Bewegungsfreiheit der Lanzette 3 durch einen Sperrbolzen 24 bewirkt, der quer zur Einstichsrichtung beweglich ist und dessen der Lanzette 3 zugewandtes Ende ein Anschlagelement 25 bildet, das den Bewegungsweg der Lanzette 3 während der Spannbewegung des Lanzettenantriebs 10 in Einstichrichtung (indirekt über den Lanzettenhalter 4) begrenzt. Der Sperrbolzen 24 bildet zugleich den Auslöser 26 des Lanzettenantriebs.

Die Bewegungsfreiheit des Lagerteils 20 innerhalb der Lagerteilführung 21 ist in Einstichrichtung (nach vorne) durch einen Anschlag 28 und entgegen der Einstichrichtung (nach hinten) elastisch durch eine Druckfeder 29 limitiert, die sich einerseits gegen das Lagerteil 20 und andererseits gegen ein (zumindest entgegen der Einstichrichtung) gehäusefest fixiertes Bauteil 30 abstützt. Die Druckfeder 29 steht vorzugsweise unter Vorspannung, übt also auch dann noch eine Druckkraft aus, wenn sie sich in der Position ihrer maximalen Ausdehnung (Bewegungspositionen a, c und d) befindet.

Die Spannbewegungsphase des Lanzettenantriebs 10 beginnt mit einer ersten geknickten Position a und führt über eine gestreckte Position b in eine zweite geknickte Position c. Dabei wird mittels eines in Figur 1 nicht dargestellten Bewegungsmechanismus eine Spannkraft F_{S} auf den Mehrhebel-Kopplungsmechanismus 12 ausgeübt, die größer ist als die entgegengerichtete Kraft F_{A} des Antriebselementes 11. Das Antriebselement kann beispielsweise von einer Blattfeder 31 gebildet werden, die der Übersichtlichkeit halber nur in Figur 1 (a) dargestellt ist, selbstverständlich aber in jeder Bewegungsphase mit einer Antriebskraft F_{A} gegen den Mehrhebel-Kopplungsmechanismus drückt.

Während der Spannbewegungsphase ist die Bewegungsfreiheit der Lanzette 3 durch den Anschlag 25 des Auslösers 26 in Einstichrichtung begrenzt. Die Bewegung von a nach b führt dazu, daß das Lagerteil 20 entgegen der Kraft der Druckfeder 29 in der der Einstichrichtung entgegengesetzten Richtung verschoben wird. Bei der weiteren Bewegung nach c entspannt sich die Druckfeder 29 wieder und der Lanzettenantrieb 10 befindet sich am Ende dieser Bewegung im gespannten Zustand (Bewegungsposition c).

Zum Auslösen der Einstichbewegung wird der Auslöser 26 aus der Bewegungsbahn des Lanzettenhalters 4 herausgezogen, so daß der Lanzettenantrieb, angetrieben durch die Antriebskraft F_{A} des Antriebselementes 11, eine Bewegung von der Position c über die Position d (maximale Einstichtiefe) und weiter in die Ausgangsposition a ausführt. Dabei tritt die Spitze 8 der Lanzette 3 aus einer (nur bei den Bewegungspostionen c und d dargestellten) Austrittsöffnung 23 aus. Die Austrittsöffnung 23 ist von einer Kontaktfläche 27 umgeben, die bei der Benutzung des Geräts gegen das Körperteil, aus dem Blut entnommen werden soll, gedrückt wird.

Während der Einstichbewegungsphase sollte sich das Lagerteil 20 in einer definierten Position befinden, um eine exakt reproduzierbare Einstichtiefe zu garantieren. Die Kraft der Druckfeder 29 ist deshalb vorzugsweise so bemessen, daß das Lagerteil 20 mindestens in der Bewegungsposition der maximalen Einstichtiefe (d in Figur 1) gegen den seine Bewegung in Einstichrichtung limitierten Anschlag 28 gedrückt wird.

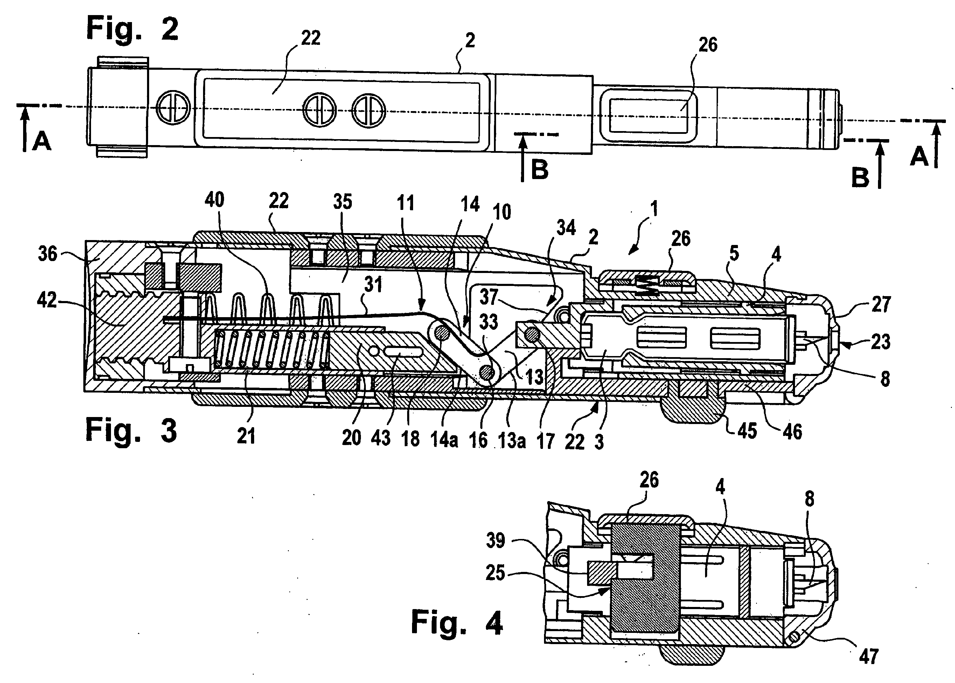

Die Figuren 2 bis 4 zeigen konstruktive Einzelheiten eines zur Erprobung der Erfindung verwendeten Blutentnahmesystems. Dabei werden die Hebel 13 und 14 jeweils durch zwei parallel verlaufende Metallstäbe 13a, 14a gebildet. Von diesen ist in Figur 3 jeweils ein vorderer zu sehen, der den hinteren abdeckt. Das Schwenkgelenk 16 wird durch einen durchgehenden Bolzen 33 gebildet, der in entsprechende Bohrungen der Stäbe 13a,14a eingreift und zwischen diesen eine Ausnehmung 34 eines Spannschiebers 35 durchsetzt. Zum Spannen wird der Spannschieber 35 mittels eines Betätigungsknopfes 36 nach hinten gezogen, wobei der Bolzen 33 über eine schräge Fläche 37 der Ausnehmung 34 gleitet und dadurch (in Figur 3 nach oben) in die gespannte Position bewegt wird. Im gespannten Zustand greift auch hier ein mit dem Auslöser 26 gekoppeltes Anschlagelement 25 in ein entsprechendes Gegenstück 39 des Lanzettenhalters 4 ein (Figur 4) und limitiert dadurch die Bewegung der Lanzette 3 in Einstichrichtung so, daß die Lanzettenspitze 8 während der Spannbewegungsphase des Lanzettenantriebs 10 nicht aus der von der Kontaktfläche 27 umgebenen Austrittsöffnung 23 austreten kann. Der Spannschieber 35 wird durch eine Flachfeder 40 in die Ausgangsposition zurückgedrückt.

Bei der dargestellten bevorzugten Ausführungsform läßt sich die Einstichtiefe durch Drehen des Betätigungsknopfes 36 einstellen. Ein Innengewinde des Betätigungsknopfes 36 wirkt mit einem Außengewinde zusammen, das am Umfang eines Schlittenteils 42 angebracht ist, welches mit der Lagerteilführung 21 verbunden ist. An der Lagerteilführung 21 ist ein nicht dargestellter Stift befestigt, der in ein entsprechendes Langloch 43 des Lagerteils 20 eingreift und dadurch den maximalen Verschiebungsweg des Lagerteils 20 in Einstichrichtung und entgegen der Einstichrichtung begrenzt. Beim Drehen des Betätigungsknopfes 36 führt die Verschiebung der Lagerteilführung 21 zu einer entsprechenden Verschiebung des gesamten Lanzettenantriebs 10 und damit zu einer Änderung der Einstichtiefe.

Bei den bisher bekannten Lanzettenantrieben bestand nicht die Möglichkeit, die Einstichtiefe dadurch zu verstellen, daß man den Kopplungsmechanismus, der die Antriebsfeder mit der Lanzette bzw. dem Lanzettenhalter verbindet, insgesamt verschiebt und die entsprechende Verstellmechanik an dem von der Austrittsöffnung abgewandten Ende des Stechgerätes vorsieht. Die Einstellung der Einstichtiefe war in der Praxis nur dadurch möglich, daß man die Position der vorderen Kappe des Stechgeräts, an der sich die Kontaktfläche 27 befindet, in Einstichrichtung einstellbar machte. Dies führte zu einer konstruktiv schwer beherrschbaren Konzentration von Funktionen im vorderen Bereich des Stechgerätes.

Bei der erfindungsgemäßen Konstruktion besteht hingegen die Möglichkeit, die Funktionen "Halterung der Lanzette" und "Einstellung der Einstichtiefe" räumlich zu trennen sind und unabhängig voneinander zu realisieren. Dies schafft Raum für zusätzliche vorteilhafte Funktionen. Bei der dargestellten Ausführungsform ist beispielsweise im Bereich des Lanzettenhalters ein Schieber 45 mit einem Auswerfer 46 vorgesehen, der dazu dient, gebrauchte Lanzetten auszuwerfen. Die Kontaktfläche 27 ist Bestandteil einer klappbaren Kappe 47.

Figur 5 zeigt eine alternative Ausführungsform, die sich vor allem dadurch von den Figuren 1 bis 4 unterscheidet, daß der Mehrhebel-Kopplungsmechanismus 12 drei Hebel aufweist, nämlich außer dem lanzettenseitigen Hebel 13 und dem antriebsseitigen Hebel 14 einen Verbindungshebel 50. Zur Kopplung der Hebel sind insgesamt fünf Schwenkgelenke erforderlich, wobei statt des einen zur Verbindung der Hebel 13 und 14 dienenden Schwenkgelenks 16 hier drei Schwenkgelenke 51 bis 53 an dem Verbindungshebel vorgesehen sind, von denen die Schwenkgelenke 51 und 53 zur Verbindung mit den Hebeln 13 und 14 dienen und das mittlere Schwenkgelenk 52 an einem zweiten beweglichen Lagerteil 54 befestigt ist, das ebenso wie das Lagerteil 20 beim Spannvorgang entgegen der Einstichrichtung beweglich ist. Durch die Verwendung von mehr als zwei Hebeln wird bei einer vorgegebenen Länge des Einstichweges die Auslenkung der Verbindungsgelenke 51 und 53 zwischen den beiden geknickten Positionen (a) und (c) reduziert und dadurch eine schlankere Bauform des Gehäuses möglich.

Die Bewegungspositionen (a) bis (c) entsprechen den mit gleichen Buchstaben bezeichneten Bewegungspositionen der Figur 1. Eine gesonderte Erläuterung ist deshalb nicht erforderlich. Eine konstruktive Besonderheit besteht darin, daß die Limitierung der Bewegungsfreiheit des Lagerteils 20 während der Einstichbewegung nicht mittels einer elastisch nachgebenden Feder, sondern mittels eines Anschlagelementes 55 erfolgt, das in eine entsprechende Ausnehmung 56 des Lagerteils 20 eingreift, wenn sich der Lanzettenantrieb in der gespannten Bewegungsposition c befindet. Der Einstichvorgang wird dann durch Druck auf den Auslöser 26 ausgelöst, der über eine Sperrklinke 57 mit dem Anschlagelement 25 in Verbindung steht, das während des Spannvorganges die Bewegungsfreiheit der Lanzette 3 in Einstichrichtung limitiert.

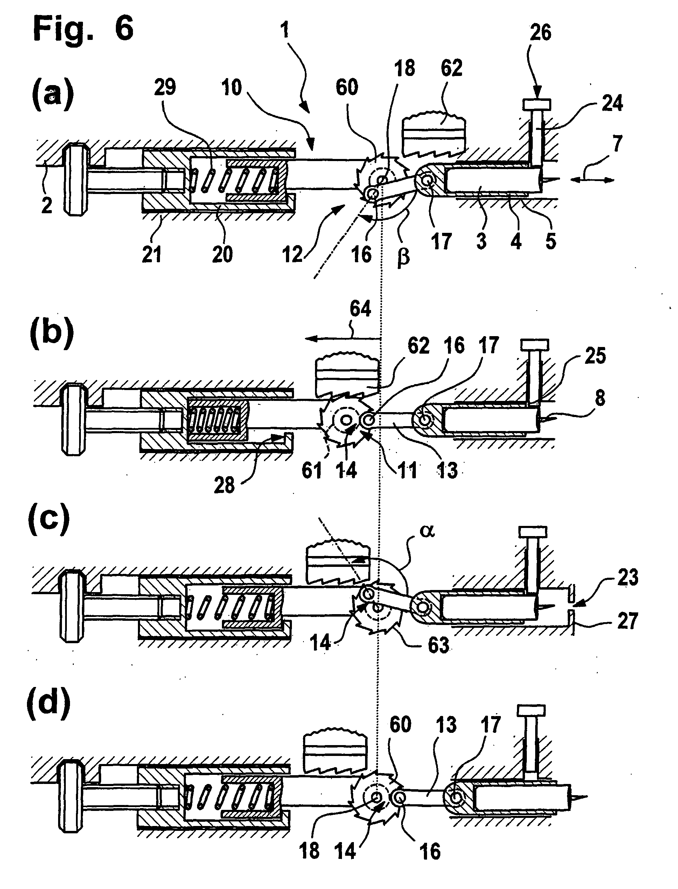

Figur 6 zeigt eine weitere Ausführungsform eines Blutentnahmesystems in einer zu Figur 1 analogen Darstellung, d.h. ebenso wie bei Figur 1 sind folgende Bewegungspositionen dargestellt:
(a) Lanzettenantrieb 10 entspannt; Mehrhebel-Kopplungsmechanismus 12 in der zweiten geknickten Position;
(b) Lanzettenantrieb 10 während des Spannvorgangs; Mehrhebel-Kopplungsmechanismus 12 in gestreckter Position;
(c) Lanzettenantrieb 10 gespannt; Mehrhebel-Kopplungsmechanismus in der ersten geknickten Position;
(d) Lanzettenantrieb 10 in der Einstichbewegung; Mehrhebel-Kopplungsmechanismus in der der maximalen Einstichtiefe entsprechenden gestreckten Position.

Übereinstimmende Bauteile sind mit den gleichen Bezugsziffern wie in Figur 1 bezeichnet und werden nicht nochmals erläutert.

Abweichend von den Figuren 1 bis 4 wird der antriebsseitige Hebel 14 des Kopplungsmechanismus 12 nicht von einem stabförmigen Bauelement, sondern von einem Rotorteil 60 gebildet, das um die Schwenkachse 18 drehbar ist. Der Winkelabstand α der Schwenkbewegung des von dem Rotorteil 60 gebildeten zweiten Hebels 14 zwischen dem gespannten Zustand c und dem gestreckten Zustand b bzw. d beträgt mehr als 90°, im dargestellten Fall etwa 135°. Auch der Winkelabstand β zwischen der gestreckten Position b, d und der entspannten Position a ist größer als 90°.

Das für die Schwenkbewegung sowohl beim Spannen des Lanzettenantriebs 10 als auch während der Einstichbewegung erforderliche Drehmoment muß mittels eines für diesen großen Schwenkwinkel geeigneten Bauelements aufgebracht werden. Als Antriebselement 11 kann insbesondere eine Drehfeder 61 verwendet werden, die in Figur 6 hinter dem Rotorteil 60 angeordnet und deswegen nur in der Teilfigur b gestrichelt dargestellt ist.

Zum Spannen eignet sich insbesondere ein in Längsrichtung des Stechgerätes bewegliches Spannelement 62, das während seiner Bewegung nach hinten (d.h. entgegen der Einstichrichtung) mit dem antriebsseitigen Hebel 14 so gekoppelt wird, daß das zum Spannen erforderliche Drehmoment auf den (von dem Rotorteil 60 gebildeten) Hebel 14 ausgeübt wird. Bei der dargestellten Ausführungsform ist dies dadurch realisiert, daß das Rotorteil 60 auf seiner Außenseite einen Zahnkranz 63 aufweist, in den entsprechende zahnförmige Klinken an der Unterseite des verschiebbaren Spannelementes 62 eingreifen, wenn dieses in der in der Teilfigur b mit dem Pfeil 64 bezeichneten Richtung nach hinten geschoben wird. Selbstverständlich kann diese Freilauffunktion auch mit einer anderen für diesen Zweck bekannten Konstruktion verwendet werden. Das Spannelement 62 ist gegen die Kraft einer nicht dargestellten Feder in Querrichtung so weit beweglich, daß es bei der Rückführung von der in der Teilfigur d dargestellten Position in die Position gemäß Teilfigur a über den Zahnkranz 63 rutscht, ohne das Rotorteil 60 zu drehen.

Dieses Ausführungsbeispiel verdeutlicht, daß die Hebel 13,14 nicht notwendigerweise durch stangenförmige Elemente gebildet werden müssen, sondern beispielsweise auch ein rotierendes Teil verwendet werden kann, das die Eigenschaften eines Hebels im weiter oben erläuterten Sinn aufweist. Die in Figur 6 dargestellte Ausführungsform eines Mehrhebel-Kopplungsmechanismus 12 ähnelt einem Kurbelantrieb. Er zeichnet sich unter anderem dadurch aus, daß bei gegebener maximaler Breite des Antriebs eine relativ große Einstichtiefe realisert werden kann.

## Patentansprüche

1. Blutentnahmesystem zur Entnahme von Blut für Diagnosezwecke, umfassend
ein Gehäuse (22) mit einer Austrittsöffnung (23) für die Spitze (8) einer Lanzette (3), die in dem Gehäuse (22) entlang einem vorbestimmten Einstichweg (7) beweglich ist,
eine Lanzettenführung (5), von der die Lanzette (3) auf dem vorbestimmten Einstichweg (7) geführt wird und
einen Lanzettenantrieb (10), durch den nach Betätigung eines Auslösers (26) eine Bewegung eines Antriebselementes (11) in eine Einstichbewegung umgesetzt wird, bei der die Lanzette (3) mit hoher Geschwindigkeit entlang dem vorbestimmten Einstichweg (7) in Einstichrichtung bewegt wird, bis ihre Spitze (8) aus der Austrittsöffnung (23) austritt,
wobei
der Lanzettenantrieb (10) einen Mehrhebel-Kopplungsmechanismus (12) aufweist, der während der Einstichbewegung eine Verbindung zwischen dem Antriebselement (11) und der Lanzette (3) bildet und zwei Hebel (13,14) einschließt, die über ein erstes Schwenkgelenk (16) miteinander verbunden sind,
einer der Hebel (13) über ein zweites Schwenkgelenk (17) mit der Lanzette (3) gekoppelt ist und der zweite Hebel (14) an seinem von der Lanzette (3) abgewandten Ende ein drittes Schwenkgelenk (18) aufweist und
der Mehrhebel-Kopplungsmechanismus (12) während der Einstichbewegung aus einer ersten geknickten Position über eine der maximalen Einstichtiefe entsprechende gestreckte Position in eine zweite geknickte Position bewegt wird und während einer Spannbewegung von der zweiten geknickten Position über die gestreckte Position in die erste geknickte Position bewegt wird,
**dadurch gekennzeichnet, daß**
die Bewegungsfreiheit der Lanzette (3) während der Spannbewegung derartig limitiert ist, daß die Lanzettenspitze (8) nicht aus der Austrittsöffnung (23) austritt, und
das dritte Schwenkgelenk (18) an einem beweglichen Lagerteil (20) befestigt ist, das in dem Gehäuse (22) während des Spannvorgangs eine Bewegung entgegen der Einstichrichtung ausführt und dessen Bewegungsfreiheit während der Einstichbewegung entgegen der Einstichrichtung limitiert ist.

2. Blutentnahmesystem nach Anspruch 1, **dadurch gekennzeichnet, daß** die Bewegungsfreiheit der Lanzette (3) während der Spannbewegung mittels des Auslösers (26) für die Einstichbewegung limitiert ist.

3. Blutentnahmesystem nach einem der Ansprüche 1 oder 2, **dadurch gekennzeichnet, daß** die Bewegungsfreiheit der Lanzette (3) durch ein Anschlagelement (25) limitiert ist.

4. Blutentnahmesystem nach einem der vorhergehenden Ansprüche, **dadurch gekennzeichnet, daß** die Bewegungsfreiheit des Lagerteils (20) während der Einstichbewegung mittels eines Anschlagelements (55) limitiert ist.

5. Blutentnahmesystem nach einem der vorhergehenden Ansprüche, **dadurch gekennzeichnet, daß** die Bewegungsfreiheit des Lagerteils (20) während der Einstichbewegung mittels einer Feder (29) elastisch limitiert ist.

6. Blutentnahmesystem nach Anspruch 5, **dadurch gekennzeichnet, daß** die Feder (29) unter Vorspannung steht.

7. Blutentnahmesystem nach einem der vorhergehenden Ansprüche, **dadurch gekennzeichnet, daß** der Mehrhebel-Kopplungsmechanismus (12) drei Hebel (13,14,50) aufweist.

8. Blutentnahmesystem nach einem der vorhergehenden Ansprüche, **dadurch gekennzeichnet, daß** der Winkelabstand (α) der Schwenkbewegung des zweiten Hebels (14) zwischen der ersten geknickten Position und der der maximalen Einstichtiefe entsprechenden gestreckten Position mehr als 90° beträgt.

9. Blutentnahmesystem nach Anspruch 8, **dadurch gekennzeichnet, daß** das Antriebselement eine auf den zweiten Hebel (14) wirkende Drehfeder (61) ist.

## Claims

1. Blood withdrawal system for diagnostic purposes, comprising
a housing (22) with an outlet opening (23) for the tip (8) of a lancet (3), which is movable in the housing (22) along a predetermined puncture path (7),
a lancet guide (5) for guiding the lancet (3) on the predetermined puncture path (7) and
a lancet drive (10) by which a movement of a drive element (11) is converted into a puncturing movement after the actuation of a trigger (26), whereby the lancet (3) is moved with high speed along the predetermined puncture path (7) in puncturing direction until its tip (8) protrudes from the outlet opening (23),
wherein
the lancet drive (10) comprises a plural lever coupling mechanism (12) forming a connection between the drive element (11) and the lancet (3) during the puncturing movement and comprising two levers (13, 14) connected to each other by means of a first swivel joint (16),
one of the levers (13) is coupled to the lancet (3) by means of a second swivel joint (17), and the second lever (14) comprises a third swivel joint (18) at its end facing away from the lancet (3), and
the plural lever coupling mechanism (12) is moved, during the puncturing movement, from a first bent position via a straight position corresponding to the maximum puncturing depth to a second bent position, and is moved, during a cocking movement, from the second bent position via the straight position to the first bent position,
**characterized in that**
the freedom of movement of the lancet (3) is limited during the cocking movement in such a manner that the lancet tip (8) does not protrude from the outlet opening (23), and
the third swivel joint (18) is connected to a moveable bearing part (20) which during the cocking movement moves inside the housing (22) in a direction opposite to the puncturing direction, and the freedom of movement of which is limited during the puncturing movement in the direction opposite to the puncturing direction.

2. Blood withdrawal system according to claim 1, **characterized in that** the freedom of movement of the lancet (3) is limited during the cocking movement by means of a trigger (26) for triggering the puncturing movement.

3. Blood withdrawal system according to claim 1 or 2, **characterized in that** the freedom of movement of the lancet (3) is limited by a stop element (25).

4. Blood withdrawal system according to any one of the preceding claims, **characterized in that** the freedom of movement of the bearing part (20) is limited during the puncturing movement by a stop element (55).

5. Blood withdrawal system according to any one of the preceding claims, **characterized in that** the freedom of movement of the bearing part (20) is elastically limited during the puncturing movement by a spring (29).

6. Blood withdrawal system according to claim 5, **characterized in that** the spring (29) is pretensioned.

7. Blood withdrawal system according to any one of the preceding claims, **characterized in that** the plural lever coupling mechanism (12) comprises three levers (13, 14, 50).

8. Blood withdrawal system according to any one of the preceding claims, **characterized in that** the angular separation (α) of the swivel movement of the second lever (14) between the first bent position and the straight position corresponding to the maximum puncturing depth, exceeds 90°.

9. Blood withdrawal system according to claim 8, **characterized in that** the drive element is a torsion spring (61) acting on the second lever (14).

## Revendications

1. Système de prélèvement sanguin à des fins de diagnostic, comprenant
un boîtier (22) avec un orifice de sortie (23) pour la pointe (8) d'une lancette (3) qui est mobile dans le boîtier (22) le long d'une trajectoire de piqûre prédéterminée (7),
un guidage de lancette (5) par lequel la lancette (3) est guidée sur la trajectoire de piqûre prédéterminée (7) et
un entraînement de lancette (10) grâce auquel, après l'actionnement d'un déclencheur (26), un mouvement d'un élément d'entraînement (11) est transformé en mouvement de piqûre lors duquel la lancette (3) est déplacée à une vitesse élevée le long de la trajectoire de piqûre prédéterminée (7) jusqu'à ce que sa pointe (8) sorte de l'orifice de sortie (23),
l'entraînement de lancette (10) comportant un mécanisme de couplage à leviers multiples (12) qui établit pendant le mouvement de piqûre une liaison entre l'élément d'entraînement (11) et la lancette (3) et comprend deux leviers (13, 14) qui sont reliés l'un à l'autre via une première articulation pivotante (16),
un des leviers (13) étant couplé à la lancette (3) via une deuxième articulation pivotante (17) et le deuxième levier (14) comportant à son extrémité opposée à la lancette (3) une troisième articulation pivotante (18) et
le mécanisme de couplage à leviers multiples (12) étant déplacé pendant le mouvement de piqûre d'une première position pliée dans une deuxième position pliée via une position tendue correspondant à la profondeur de piqûre maximale et, pendant un mouvement de tension, de la deuxième position pliée dans la première position pliée via la position tendue,
**caractérisé en ce que**
la liberté de mouvement de la lancette (3) pendant le mouvement de tension est limitée de telle sorte que la pointe de la lancette (8) ne sort pas de l'orifice de sotie (23) et
la troisième articulation pivotante (18) est fixée sur un élément de palier mobile (20) qui effectue dans le boîtier (22) un mouvement contraire au sens de piqûre pendant la tension et dont la liberté de mouvement pendant le mouvement de piqûre est limitée dans le sens contraire à celui de la piqûre.

2. Système de prélèvement sanguin selon la revendication 1, **caractérisé en ce que** la liberté de mouvement de la lancette (3) pendant le mouvement de tension est limitée au moyen du déclencheur (26) du mouvement de piqûre.

3. Système de prélèvement sanguin selon une des revendications 1 ou 2, **caractérisé en ce que** la liberté de mouvement de la lancette (3) est limitée par un élément de butée (25).

4. Système de prélèvement sanguin selon l'une des revendications précédentes, **caractérisé en ce que** la liberté de mouvement de l'élément de palier (20) pendant le mouvement de piqûre est limitée au moyen d'un élément de butée (55).

5. Système de prélèvement sanguin selon l'une des revendications précédentes, **caractérisé en ce que** la liberté de mouvement de l'élément de palier (20) pendant le mouvement de piqûre est limitée de manière élastique au moyen d'un ressort (29).

6. Système de prélèvement sanguin selon la revendication 5, **caractérisé en ce que** le ressort (29) est précontraint.

7. Système de prélèvement sanguin selon l'une des revendications précédentes, **caractérisé en ce que** le mécanisme de couplage à leviers multiples (12) comporte trois leviers (13, 14, 50).

8. Système de prélèvement sanguin selon l'une des revendications précédentes, **caractérisé en ce que** l'écart angulaire (α) du mouvement de pivotement du deuxième levier (14) entre la première position pliée et la position tendue correspondant à la profondeur de piqûre maximale est supérieur à 90°.

9. Système de prélèvement sanguin selon la revendication 8, **caractérisé en ce que** l'élément d'entraînement est un ressort de torsion (61) qui agit sur le deuxième levier (14).
